(19) Europäisches Patentamt
European Patent Office
Office européen des brevets

(11) Veröffentlichungsnummer: **0 124 889**
**A2**

(12) **EUROPÄISCHE PATENTANMELDUNG**

(21) Anmeldenummer: 84105025.5

(51) Int. Cl.³: C 07 D 501/46

(22) Anmeldetag: 04.05.84

(30) Priorität: 07.05.83 DE 3316798

(43) Veröffentlichungstag der Anmeldung:
14.11.84 Patentblatt 84/46

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Schwab, Wilfried, Dr.
Am Flachsland 18
D-6233 Kelkheim (Taunus)(DE)

(72) Erfinder: Dürckheimer, Walter, Dr.
Im Lerchenfeld 45
D-6234 Hattersheim am Main(DE)

(72) Erfinder: Kirrstetter, Reiner, Dr.
Am Flachsland 56
D-6233 Kelkheim (Taunus)(DE)

(72) Erfinder: Lattrell, Rudolf, Dr.
Heuhohlweg 6H
D-6240 Königstein/Taunus(DE)

(54) **Verfahren zur Herstellung von Cephemverbindungen.**

(57) Verfahren zur Herstellung von Cephemverbindungen der Formel

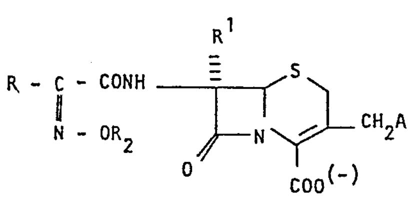

EP 0 124 889 A2

0124889

## Verfahren zur Herstellung von Cephemverbindungen

Gegenstand der Erfindung ist ein Verfahren zur Herstellung von Cephemverbindungen der allgemeinen Formel I,

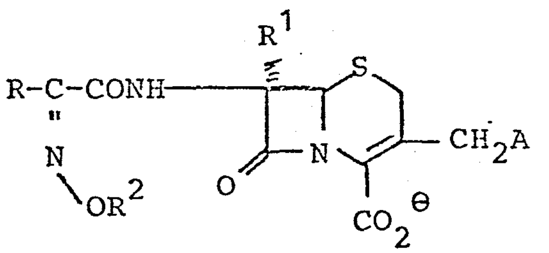

(I)

worin R einen Thiazolylrest

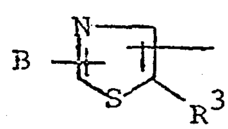

oder einen 1,2,4-Thiadiazolylrest

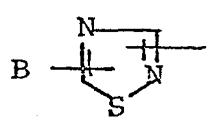

bedeutet, in denen $R^3$ für Wasserstoff oder Halogen und B für eine gegebenenfalls substituierte Aminogruppe steht, und worin

$R^1$ Wasserstoff oder Methoxy

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $(CH_2)_n \overset{R^4}{\underset{R^5}{(C)}}_m R^6$, worin m und n jeweils für O oder 1 steht,

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und die $C_3$-$C_7$-Cycloalkylidengruppe noch weiter ein- oder mehrfach substituiert sein können,

$R^6$ eine COOH-; CN- oder $CONH_2$-Gruppe, wobei letztere am Stickstoff ein oder zweimal substituiert sein kann,

A einen Chinolinium 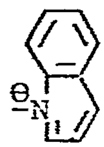 oder einen Isochinoliniumrest 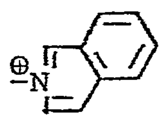, die jeweils auch ein- oder mehrfach, gleich oder verschieden substituiert sein können durch gegebenenfalls substituiertes $C_1$-$C_6$-Alkyl, $C_1$-$C_6$-Alkoxy, Halogen, Trifluormethyl oder Hydroxy, oder einen Phenanthridiniumrest, oder einen

Pyridiniumrest 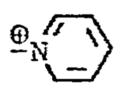, der ein- oder mehrfach, gleich oder verschieden substituiert sein kann durch gegebenenfalls substituiertes $C_1$-$C_6$-Alkyl, wobei 2 orthoständige Alkylgruppen auch zu einem gegebenenfalls substituierten Di- bis Decamethylen-Ring geschlossen sein können, in dem ein Ring-C-Atom durch ein Heteroatom ersetzt sein kann und weiterhin auch noch eine oder zwei Doppelbindungen enthalten sein können; durch gegebenenfalls substituiertes $C_2$-$C_6$-Alkenyl, durch $C_2$-$C_6$-Alkinyl, durch $C_3$-$C_7$-Cycloalkyl oder $C_3$-$C_7$-Cycloalkylmethyl, wobei in beiden Substituenten der Ring auch substituiert sein kann; durch $C_4$-$C_7$-Cycloalkenyl, durch gegebenenfalls substituiertes $C_1$-$C_6$-Alkoxy, durch $C_2$-$C_6$-Alkenyloxy oder $C_2$-$C_6$-Alkinyloxy, durch Halogen, Trifluormethyl oder

Hydroxy, durch gegebenenfalls substituiertes Phenyl, Benzyl oder Heteroaryl,

durch Formyl oder ketalisiertes Formyl,

durch gegebenenfalls substituiertes $C_1$-$C_6$-Alkylcarbonyl, das auch in ketalisierter Form vorliegen kann,

durch Arylcarbonyl,

durch Carbamoyl,

bedeutet und in der die $R^2O$-Gruppe in syn-Position steht.

Die vorliegende Erfindung ist insbesondere auf Verbindungen gerichtet, in denen R und $R^1$ die vorstehenden Bedeutungen besitzen und

B eine Aminogruppe, die substituiert sein kann durch Aminoschutzgruppen,

$R^2$ Wasserstoff, $C_1$-$C_6$-Alkyl, das ein- oder mehrfach substituiert sein kann durch Halogen, $C_1$-$C_6$-Alkylthio, $C_1$-$C_6$-Alkyloxy, Aryl oder Heteroaryl, $C_2$-$C_6$-Alkenyl, das ein- oder mehrfach substituiert sein kann durch Halogen; $C_2$-$C_3$-Alkinyl, $C_3$-$C_6$-Cycloalkyl, $C_3$-$C_6$-Cycloalkyl-$C_1$-$C_6$-alkyl, $C_4$-$C_7$-Cycloalkenyl und in der die Gruppe $(CH_2)_n\underset{R^5}{\overset{R^4}{(\overset{|}{\underset{|}{C}})}}_m R^6$ die vorstehende Bedeutung hat,

A einen Chinolinium- oder einen Isochinoliniumrest, die jeweils auch ein- oder mehrfach, gleich oder verschieden substituiert sein können

durch $C_1$-$C_6$-Alkyl, das substituiert sein kann durch Hydroxy;

durch $C_1$-$C_6$-Alkoxy,

durch Halogen,

durch Trifluormethyl,

durch Hydroxy, oder

einen Pyridiniumrest 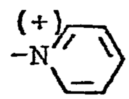 bedeutet, der ein- oder mehrfach, gleich oder verschieden substituiert sein kann

durch $C_1$-$C_6$-Alkyl, das ein- oder mehrfach substituiert sein kann durch Hydroxy; Formyl oder $C_1$-$C_6$-Alkylcarbonyl, deren Carbonylgruppen auch in ketalisierter Form vorliegen können, Sulfo, Carbamoyl, $C_1$-$C_6$-Alkyloxy, Hydroxy-$C_1$-$C_6$-alkyloxy und wobei 2 Alkylgruppen auch zu einem gegebenenfalls substituierten Di- bis Decamethylen-Ring geschlossen sein können, in dem ein Ring-C-Atom durch ein Heteroatom ersetzt sein kann und weiterhin auch noch eine oder zwei Doppelbindungen enthalten sein können,

durch $C_2$-$C_6$-Alkenyl, das durch Hydroxy substituiert sein kann,

durch $C_2$-$C_6$-Alkinyl

durch $C_3$-$C_7$-Cycloalkyl oder $C_3$-$C_7$-Cycloalkylmethyl, wobei in beiden Substituenten der Ring auch substituiert sein kann durch Hydroxy oder Halogen,

0124889

durch $C_4$-$C_7$-Cycloalkenyl

durch $C_1$-$C_6$-Alkoxy, das durch Hydroxy substituiert sein kann,

durch $C_2$-$C_6$-Alkenyloxy oder $C_2$-$C_6$-Alkinyloxy,

durch Halogen, Trifluormethyl oder Hydroxy,

durch Phenyl, Benzyl oder Heteroaryl, die auch durch Halogen substituiert sein können,

durch Formyl oder ketalisiertes Formyl,

durch $C_1$-$C_6$-Alkylcarbonyl, das auch durch Hydroxy substituiert sein und auch in ketalisierter Form vorliegen kann,

durch Arylcarbonyl,

durch Carbamoyl,

und wobei auch bei diesen bevorzugten, unter die allgemeine Formel I fallenden Verbindungen die $R^2O$-Gruppe in syn-Position steht.

Als gegebenenfalls mögliche Substituenten des unter A erwähnten Di- bis Decamethylen-Rings, in dem ein Ring-C-Atom durch ein Heteroatom ersetzt sein kann und weiterhin auch noch eine oder zwei Doppelbindungen enthalten sein können, kommen insbesondere die folgenden Substituenten in Betracht, die ein- oder mehrfach, vorzugsweise jedoch einfach auftreten können:

$C_1$-$C_6$-Alkyl, $C_1$-$C_4$-Alkoxy, Hydroxymethyl, Halogen, Hydroxy, Oxo, Exomethylen.

Diese Substituenten können an den genannten, an den Pyridiniumrest ankondensierten Ringen auftreten, unabhängig davon, ob der jeweilige Ring gesättigt, ungesättigt oder auch noch durch ein Heteroatom unterbrochen ist.

Der an den Pyridiniumrest ankondensierte Ring kann 2 bis 10 Ringglieder (Di- bis Decamethylen), vorzugsweise jedoch 3 bis 5 Ringglieder enthalten und somit beispielsweise einen Cyclopenteno-, Cyclohexeno- oder Cyclohepteno-Ring darstellen. Enthält ein solcher ankondensierter Ring eine Doppelbindung, so seien als Beispiele ein Dehydrocyclopentadieno-, Dehydrocyclohexadieno- oder Dehydrocycloheptadieno-Ring genannt. Ist in derartigen Ringen ein C-Atom durch ein Heteroatom ersetzt, so kommen als Heteroatome insbesondere Sauerstoff oder Schwefel in Betracht. Als ein Sauerstoffatom enthaltende, ankondensierte Ringe, die zwei oder eine Doppelbindung enthalten, seien beispielsweise erwähnt Furo, Pyrano, Dihydrofuro und Dihydropyrano; als ankondensierte Ringe mit einem Schwefelatom, die zwei oder eine Doppelbindung enthalten Thieno, Thiopyrano, Dihydrothieno und Dihydrothiopyrano. Von den ein Heteroatom enthaltenden, ankondensierten Ringen kommen für eine Substitution, insbesondere durch die vorstehend angegebenen Substituenten, insbesondere diejenigen Ringe in Betracht, die nur eine Doppelbindung enthalten.

Als besonders bevorzugt kommen beispielsweise die folgenden Substituenten in Betracht:

B: $NH_2$, HCONH, $CF_3CONH$, $CCl_3CONH$, $C_6H_5CH_2CONH$, $(C_6H_5)_3CNH$ $HSO_3NH$, $(CH_3)_2$ CH=N,

R: 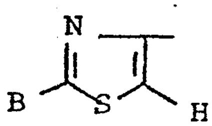 , 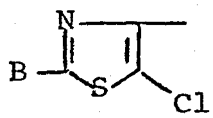 , 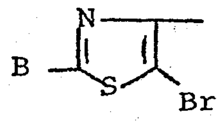 ,

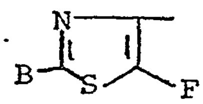 , 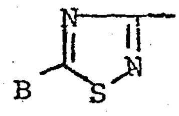

$R^1$: Wasserstoff, $OCH_3$

$R^2$: Wasserstoff, $C_1$-$C_6$-Alkyl, wie z.B. Methyl, Ethyl, Propyl, Isopropyl, Butyl, vorzugsweise Methyl, Ethyl; $C_1$-$C_2$-Halogenalkyl, z.B. Chlor, Brom, Jod oder Fluorsubstituiertes Alkyl, vorzugsweise Trifluorethyl, Difluormethyl, 2,2,3,3-Tetrafluorpropyl, durch Aryl, wie z.B. Phenyl, Tolyl, Chlorphenyl substituiertes Alkyl, insbesondere Benzyl,
durch Heteroaryl substituiertes Alkyl, wie z.B. 1,3-Thiazol-4-yl-substituiertes Alkyl, insbesondere 1,3-Thiazol-4-yl-methyl,
$C_2$-$C_6$-Alkenyl, wie z.B. Vinyl, Allyl, Isopropenyl, Methallyl, insbesondere Allyl, Methallyl,
durch Halogen, wie z.B. durch Chlor oder Brom substituiertes $C_2$-$C_6$-Alkenyl, insbesondere 3-Chlor-propen-2-yl, 2-Brom-propen-2-yl, 2-Chlorpropen-2-yl;
$C_2$-$C_3$-Alkinyl, wie insbesondere Propargyl,
$C_3$-$C_7$-Cycloalkyl, wie insbesondere Cyclopropyl, Cyclobutyl, Cyclopentyl,
$C_3$-$C_7$-Cycloalkyl-$C_1$-$C_6$-alkyl, wie insbesondere Cyclopropylmethyl, Cyclobutylmethyl
$C_4$-$C_7$-Cycloalkenyl, wie insbesondere Cyclopenten-1-yl,
die Gruppe $(CH_2)_n(\overset{R^4}{\underset{R^5}{C}})_m-R^6$ wobei $R^4$ und $R^5$ gleich oder

verschieden sein können und Wasserstoff, Aryl, vorzugsweise Phenyl, $C_1$-$C_4$-Alkyl, wie z.B. Methyl, Ethyl, Propyl, Isopropyl, Butyl, sec. Butyl, vorzugsweise Methyl, Ethyl, insbesondere Methyl, bedeuten können, oder
wobei $R^4$ und $R^5$ zusammen mit dem Kohlenstoffatom, an das sie gebunden sind, eine Methylengruppe oder eine $C_3$-$C_7$-Cycloalkylidengruppe bilden können, wie z.B. Cyclopropyl, Cyclobutyl, Cyclopentyl, Cyclohexyl, Cycloheptyl, vorzugsweise Cyclopropyl, Cyclobutyl, Cyclopentyl oder Cyclohexyl, und wobei die Cycloalkylidengruppe substituiert sein kann, z.B. durch $C_1$-$C_4$-Alkyl, vorzugsweise Methyl, durch Halogen, vorzugsweise Fluor und Chlor, oder auch substituiert sein kann durch Alkylen mit 3 - 6 C-Atomen,

m = 0 oder 1
n = 0 oder 1, wobei die Summe von m und n 1 oder 2 darstellt.

Bevorzugte Beispiele für die Gruppe $-(CH_2)_n(\overset{R^4}{\underset{R^5}{C}})_m-$ sind die folgenden:

Für den Fall, daß n = 0 und m = 1 ist;

$-CH(CH_3)$, $-C(CH_3)_2$, $-CH(C_6H_5)$,

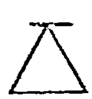 , 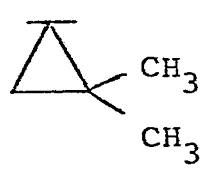 , 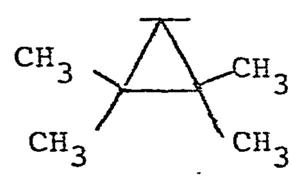 , 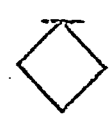

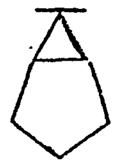 , 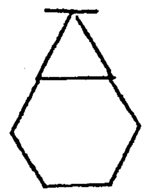 , 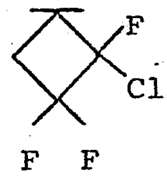 , 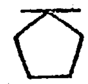 , 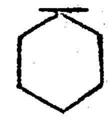

für den Fall, daß m = 0 und n = 1 ist: $-CH_2-$ und falls n und m = 1 sind: $-CH_2-C(=CH_2)-$.

$R^6$ die Gruppe COOH, CN, $CONH_2$, durch $C_1$-$C_6$-Alkyl, vorzugsweise Methyl oder Ethyl, substituiertes Carbamoyl.

A einen Chinolinium- oder einen Isochinoliniumrest, die jeweils auch ein- oder mehrfach, gleich oder verschieden substituiert sein können durch $C_1$-$C_6$-Alkyl, wie z.B. Methyl, Ethyl, Propyl, Isopropyl, vorzugsweise Methyl, durch Methoxy, durch Hydroxy, durch Halogen oder Trifluormethyl, einen Pyridiniumrest, der ein- oder mehrfach, vorzugsweise 1- bis 3-fach, insbesondere 1- bis 2-fach substituiert seinkann, beispielsweise durch $C_1$-$C_4$-Alkyl, wie insbesondere Methyl, Ethyl, Propyl, Isopropyl, n-Butyl, sec.-Butyl, tert.-Butyl, Dimethyl, Trimethyl, Methyl und Ethyl, Methyl und Propyl, Methyl und Isopropyl, Ethyl und Ethyl; Hydroxy-$C_1$-$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei z.B. auch zwei oder drei Hydroxygruppen am Alkylrest stehen können; Formyl-$C_1$-$C_4$-alkyl, wie insbesondere Formylmethyl, $C_1$-$C_4$-Alkyl-carbonyl-$C_1$-$C_4$-alkyl, wie insbesondere Methylcarbonylmethyl, Ethylcarbonylmethyl, Methylcarbonylethyl und Ethylcarbonylethyl; $C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl; $C_3$-Alkinyl, wie

0124889

insbesondere Propargyl; $C_3$-$C_6$-Cycloalkyl und $C_3$-$C_6$-Cycloalkyl-methyl, wobei sich die Kohlenstoffzahl auf den Cycloalkylteil bezieht, wie insbesondere Cyclopropyl, Cyclobutyl, Cyclopentyl, Cyclohexyl und Cyclopentylmethyl, wobei die Ringe auch substituiert sein können, z.B. durch Hydroxy, wie insbesondere 1-Hydroxy-1-cyclopentyl und 1-Hydroxy-1-cyclohexyl, oder durch Halogen, vorzugsweise Chlor;

$C_5$-$C_6$-Cycloalkenyl, wie insbesondere Cyclopenten-1-yl und Cyclohexen-1-yl;

$C_1$-$C_6$-Alkoxy, wie insbesondere Methyl-und Ethyloxy, Halogen, wie insbesondere 3-Fluor, 3-Chlor, 3-Brom, 3-Jod; Hydroxy; insbesondere 3-Hydroxy; Trifluormethyl, insbesondere 3-Trifluormethyl; Phenyl und Benzyl, die auch substituiert sein können, beispielsweise durch Halogen, insbesondere Chlor, wie z.B. 4-Chlorbenzyl; 2'-Thienyl und 3'-Thienyl;

$C_1$-$C_4$-Alkylcarbonyl, insbesondere Acetyl und Propionyl, vorzugsweise Acetyl; Formyl, Benzoyl, Carbamoyl.

Stellt A einen Pyridiniumrest dar, der durch zwei zu einem Di- bis Decamethylen-Ring geschlossene Alkylgruppen substituiert ist, der wiederum ein- oder mehrfach, vorzugsweise einfach substituiert sein und eine oder zwei Doppelbindungen enthalten kann, so kommen hierfür ganz besonders die folgenden ankondensierten Ringsysteme in Betracht:

Cyclobuteno, Cyclopenteno, Hydroxycyclopenteno, Oxocyclopenteno, Hydroxymethylcyclopenteno, Exomethylencyclopenteno, Carboxycyclopenteno und Carbamoyl-cyclopenteno,

Cyclohexeno, Hydroxycyclohexeno, Oxocyclohexeno, Hydroxymethyl-cyclohexeno, Exomethylen-cyclohexeno,

Carboxycyclohexeno und Carbamoylcyclohexeno,

Cyclohepteno, Hydroxy-, Oxo-, Hydroxymethyl-, Exomethylen-, Carboxy-cyclohepteno und Carbamoyl-cyclohepteno; Dehydro-cyclopenteno, Dehydro-cyclohexeno und Dehydro-cyclohepteno.

Ist in den vorstehend genannten ankondensierten Ringsystemen ein Ring-C-Atom durch ein Heteroatom, insbesondere Sauerstoff ersetzt, so kommen insbesondere in Betracht:
Furo[2,3-b]pyridin, Furo[3,2-b]pyridin, Furo[2,3-c]pyridin, Furo[3,2-c]pyridin, Furo[3,2-c]pyridin, Thieno[2,3-b]pyridin, Thieno[3,2-b]pyridin, Thieno[2,3-c]pyridin, Thieno[3,2-c]pyridin, Thieno[3,4-b]pyridin, Thieno[3,4-c]pyridin

Das erfindungsgemäße Verfahren zur Herstellung von Verbindungen der Formel I ist dadurch gekennzeichnet, daß man eine Verbindung der allgemeinen Formel II,

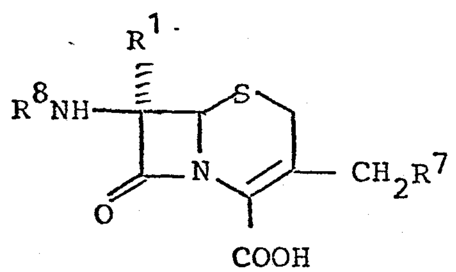

(II)

worin $R^1$ die in Formel I genannte Bedeutung hat

$R^7$ eine durch diejenige Base, die den Resten A der Formel I entspricht, austauschbare Gruppe bedeutet, und

$R^8$ für Wasserstoff oder eine Aminoschutzgruppe steht, mit der Base, die dem in Formel I definierten Rest A zugrunde liegt und in Gegenwart von Tri-$C_1$-$C_4$-alkyljodsilan, vorzugsweise Trimethyl- oder Triethyljodsilan umsetzt unter Bildung der Verbindung der allgemeinen Formel III,

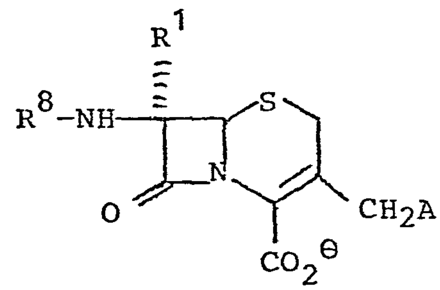

(III)

in der $R^1$, $R^8$ und A die oben genannte Bedeutung haben und

a) eine gegebenenfalls vorhandene Aminoschutzgruppe abspaltet und

b) die Verbindung III, worin $R^8$ Wasserstoff bedeutet, entweder als solche oder in Form eines reaktionsfähigen Derivates mit einer 2-syn-Oxyiminoessigsäure der allgemeinen Formel IV,

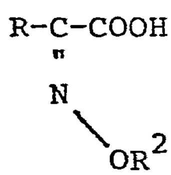

(IV)

worin R und $R^2$ die genannte Bedeutung besitzen, oder mit einem an der Carbonylgruppe aktivierten Derivat dieser Verbindung umsetzt.

Besonders bevorzugt ist die Verwendung von Trimethyljodsilan.

Die Ausgangsverbindungen sind literaturbekannt oder können nach literaturbekannten Verfahren hergestellt werden (vgl. z.B. DE-OS 27 16 707, DE-OS 31 18 732, deutsche Patentanmeldungen P 32 07 840, P 32 47 613, P 32 47 614).

Als Reste $R^7$ kommen insbesondere in Betracht Acyloxyreste von niederen aliphatischen Carbonsäuren, vorzugsweise mit 1 bis 4 C-Atomen, wie z.B. Acetoxy oder Propionyloxy, insbesondere Acetoxy, die gegebenenfalls substituiert sein können, wie z.B. Chloracetoxy oder Acetylacetoxy. Für $R^7$ kommen auch andere Gruppen in Betracht, wie beispielsweise Carbamoyloxy.

Es ist aus EP 64 740 sowie P 32 07 840.4 bekannt, daß Verbindungen der allgemeinen Formel I, in der R einen 2-Aminothiazol-4-yl-Rest bedeutet, und ihre physiologisch verträglichen Säureadditionssalze ausgezeichnete antibakterielle Wirksamkeit besitzen. Diese Verbindungen lassen sich beispielsweise aus Verbindungen der allgemeinen Formel II über die Verbindungen der allgemeinen Formel III, die aus II durch direkte Umsetzung mit den entsprechenden Basen, vorzugsweise in Wasser oder wäßrigen Gemischen als Lösungsmittel, erhalten werden, und anschließende Acylierung mit den Säuren der Formel IV herstellen.

Es wurde nun gefunden, daß nach dem erfindungsgemäßen Verfahren die Verbindungen der allgemeinen Formel I in überraschend hoher Ausbeute erhalten werden, wenn die nucleophile Austauschreaktion an Verbindungen der allgemeinen Formel II so vorgenommen wird, daß die Reaktion von Beginn an in Gegenwart eines Überschusses der entsprechenden, dem Rest A in Formel I zugrundeliegenden Basen und von Tri-$C_1$-$C_4$-alkyl- vzw. Trimethyljodsilan vorgenommen wird und die gebildeten Verbindungen der allgemeinen Formel III anschließend mit Verbindungen der allgemeinen Formel IV acyliert werden.

Das erfindungsgemäße Verfahren wird so durchgeführt, daß zu einer Lösung oder Suspension der Verbindung II in einem geeigneten Lösungsmittel die dem Rest A entsprechende

Base zugegeben wird, gefolgt von Trimethyljodsilan. Statt Trimethyljodsilan kann beispielsweise auch eine Reaktionsmischung aus Jod und Hexamethyldisilan, die vorher bei Temperaturen zwischen etwa 60 und 120° in literaturbekannter Weise zur Reaktion gebracht wurden, wobei Trimethyljodsilan entsteht, verwendet werden. Statt Trimethyljodsilan kann mit demselben guten Ergebnis auch Triethyljodsilan, das in literaturbekannter Weise hergestellt wird, verwendet werden.

Die Reaktion wird ausgeführt bei Temperaturen zwischen etwa -5° und +100°C, vorzugsweise zwischen +10° und +80°C.

Geeignete inerte aprotonische Lösungsmittel sind z.B. chlorierte Kohlenwasserstoffe, wie Methylenchlorid, Chloroform, Dichlorethan, Trichlorethan, Tetrachlorkohlenstoff, oder Niederalkylnitrile, wie Acetonitril oder Propionitril oder Frigene; insbesondere ist Methylenchlorid ein hervorragendes Lösungsmittel.

Die dem Rest A entsprechende Base wird in mindestens stöchiometrischer Menge bis zu einem zwanzigfachen Überschuß zugegeben, vorzugsweise wird mit solchen Mengen gearbeitet, daß die freiwerdende Jodwasserstoffmenge gebunden wird und noch mindestens 1 Mol, vorzugsweise 2-5 Mol der Base für die Substitution zur Verfügung stehen.

Da neben der auszutauschenden Gruppe $R^7$ in der Ausgangsverbindung II auch andere funktionelle Gruppen, wie z.B. die Carboxylgruppe, mit Trimethyljodsilan reagieren, wird letzteres in mindestens doppeltem bis zu etwa fünfzehn-fachem, vorzugsweise in drei- bis zehnfachem Überschuß zugegeben.

Derartige funktionelle Gruppen können auch durch Zugabe eines Silylierungsmittels wie z.B. Bistrimethylsilyl-

acetamid, Bistrimethylsilyltrifluoroacetamid, Trimethylchlorsilan, Hexamethyldisilazan, Bistrimethylsilylharnstoff vorsilyliert werden, entweder ohne oder in Gegenwart einer Base, vorzugsweise der gewünschten, der Gruppe A zugrundeliegenden Base in den vorstehend beschriebenen Mengen. Anschließend wird Trimethyljodsilan in mindestens stöchiometrischer Menge oder auch im Überschuß, vorzugsweise in einem doppelten bis zu einem zehnfachen Überschuß zugegeben.

Wenn in der dem Rest A in Formel I zugrundeliegenden Base funktionelle Gruppen, wie z.B. Hydroxygruppen und dergleichen vorliegen, so werden diese vorzugsweise mit einem der vorstehend genannten Silylierungsmittel vorsilyliert und sodann in der Reaktion eingesetzt.

Die Reaktionsprodukte der Formel III können beispielsweise durch Zugabe von Wasser oder wäßrigen Mineralsäuren, z.B. verdünnte HCl, HBr, HJ oder $H_2SO_4$ aus der wäßrigen Phase in üblicher Weise, z.B. durch Gefriertrocknen der Wasserphase, Chromatographie, Fällung durch Zugabe von organischen Lösungsmitteln oder durch Ausfällen aus der wäßrigen Lösung in Form eines schwerlöslichen Salzes, beispielsweise eines Hydrojodidsalzes, isoliert werden.

Die Verbindung der allgemeinen Formel III wird anschließend mit Carbonsäuren der allgemeinen Formel IV acyliert, wobei eine gegebenenfalls zur besseren Durchführung der Austauschreaktion vorhandene Aminoschutzgruppe $R^8$, beispielsweise eine tert. Butyl, Benzyl, Trityl, Benzhydryl, Formyl, Trichloracetyl, Trifluoracetyl, Sulfo oder Dimethylaminomethylen-Gruppe, in an sich bekannter Weise vorher abgespalten wird.

Werden die Carbonsäuren der allgemeinen Formel IV selbst als Acylierungsmittel eingesetzt, so wird zweckmäßig in

Gegenwart eines Kondensationsmittels, beispielsweise eines Carbodiimids, wie beispielsweise N,N'-Dicyclohexylcarbodiimid, gearbeitet.

Die Aktivierung der Carbonsäuren der allgemeinen Formel IV kann in besonders günstiger Weise durch Behandlung mit bestimmten Carbonsäureamiden und beispielsweise Phosgen, Phosphorpentachlorid, Tosylchlorid, Thionylchlorid oder Oxalylchlorid erfolgen, wie sie in der deutschen Patentschrift 28 04 040 beschrieben wird.

Als aktivierte Derivate der Carbonsäuren der allgemeinen Formel IV eignen sich insbesondere auch Halogenide, vorzugsweise Chloride, die in an sich bekannter Weise durch Behandlung mit Halogenierungsmitteln, wie z.B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel IV eignen sich ferner die Anhydride und gemischten Anhydride, Azide und aktivierten Ester und Thioester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol, 6-Chlor-1-hydroxybenzotriazol und 2-Mercaptobenzthiazol. Als gemischte Anhydride sind besonders geeignet solche mit niederen Alkansäuren, wie z.B. Essigsäure, und besonders bevorzugt solche mit substituierten Essigsäuren, wie z.B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV, in denen die Aminogruppe geschützt ist, mit Chlorameisensäure-benzylester, -p-nitrobenzylester,

-iso-butylester, -ethylester oder -allylester gewinnt. Die aktivierten Derivate können als isolierte Substanzen, aber auch in situ umgesetzt werden.

Im allgemeinen erfolgt die Umsetzung der Cephemderivate der allgemeinen Formel III mit einer Carbonsäure der allgemeinen Formel IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform, Ether, wie z.B. Diethylether, Tetrahydrofuran und Dioxan; Ketone, wie vorzugsweise Aceton und Butanon; Amide, wie vorzugsweise Dimethylformamid und Dimethylacetamid oder Pyridin. Es kann sich auch als vorteilhaft erweisen, Gemische der genannten Lösungsmittel zu verwenden. Dies ist oftmals dann der Fall, wenn die Cephemverbindung der allgemeinen Formel III mit einem in situ erzeugten aktivierten Derivat einer Carbonsäure der Formel IV umgesetzt wird.

Die Umsetzung von Cephemverbindungen der Formel III mit Carbonsäuren der Formel IV bzw. deren aktivierten Derivaten kann in einem Temperaturbereich von etwa -80 bis etwa +80°C vorzugsweise zwischen -30 und +50°C, insbesondere jedoch zwischen etwa -20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie

z.B. Triethylamin oder Dimethylanilin, anorganische Basen, wie z.B. Kaliumcarbonat oder Natriumcarbonat, Alkylenoxide, wie z.B. Propylenoxid.
Auch die Anwesenheit eines Katalysators, wie z.B. von Dimethylaminopyridin kann gegebenenfalls von Vorteil sein.

Liegt in den Verbindungen der allgemeinen Formel III die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel III mit einer Silylverbindung gebildet werden, wie z.B. Trimethylchlorsilan oder Bis-(trimethyl-silyl)-acetamid. Wird die Umsetzung mit einer solchen, an der Aminogruppe aktivierten Verbindung durchgeführt, so ist es zweckmäßig, die Reaktion in einem inerten Lösungsmittel, wie z.B. Methylenchlorid, Tetrahydrofuran oder Dimethylformamid durchzuführen.

Die folgenden Ausführungsbeispiele für die nach dem erfindungsgemäßen Verfahren herstellbaren Verbindungen dienen zur weiteren Erläuterung der Erfindung, schränken sie jedoch nicht darauf ein.

Beispiel 1:

7-[2-(2-Aminothiazol-4-yl)-2-syn-methoxyimino-acetamido]-3-[(2,3-cyclopenteno-1-pyridinio)methyl]-ceph-3-em-4-carboxylat-Dihydrojodid

2.72 g (10 mmol) 7-Aminocephalosporansäure werden in 160 ml trockenem Methylendichlorid suspendiert, dazu werden 7.1 ml (60 mmol) 2.3-Cyclopentenopyridin, dann 7.1 ml (50 mmol) Trimethyljodsilan gegeben und 2 Stunden unter Rückfluß erhitzt. Die rotbraun gefärbte Lösung wird auf 0° gekühlt und innerhalb von 2 Stunden portionsweise 4.77 g (15 mmol) des Aktivesters der 2-(2-Amino-1.3-thiazol-4-yl)-2-syn-methoxyimino-essigsäure mit 1-Hydroxybenzotriazol zugegeben. Es wird 17 Stunden bei 20°C gerührt und sodann eine Lösung von 25 g Kaliumjodid in 200 ml 2n HCl bei 0° zugegeben. Nach 3 Stunden bei 0° wird der Niederschlag abgesaugt, nacheinander mit Methylenchlorid, Eiswasser, Aceton und Äther gewaschen und über $P_2O_5$ i. Vak. getrocknet. Man erhält 5.35 g (68 %) der Titelverbindung in Form hellgelber Kristalle vom Zers.punkt 179 - 181°C.

$C_{22}H_{22}N_6O_5S_2$ x 2HJ x $H_2O$ (788.43)

| | | | | | | |
|---|---|---|---|---|---|---|
| Ber. | C 33.51 | H 3.32 | J 32.19 | N 10.66 | S 8.13 | $H_2O$ 2.3 % |
| Gef. | C 33.6 | H 3.6 | J 31.3 | N 10.7 | S 7.1 | $H_2O$ 2.5 % |

$I_R$ (KBr): 1785 $cm^{-1}$ (Lactam-CO)

$^1$H-NMR ($CF_3CO_2D$): $\delta$ = 2.30 - 2.85 (m, 2H, Cyclopenten-H); 3.10 - 4.05 (m, 6H, 4 Cyclopenten-H und $SCH_2$); 4.41 (s, 3H, $OCH_3$); 5.21 - 6.23 (m, 4H, $CH_2$Py und 2 Lactam-H); 8.11 (s, 1H, Thiazol); 7.65 - 8.70 ppm (m, 3H, Py).

Beispiel 2:

a) 7-Amino-3-[(2.3-Cyclopenteno-1-pyridinio)methyl]-ceph-3-em-4-carboxylat-Hydrojodid

Variante 1

Zu einer Suspension von 13.6 g (0.05 mol) 7-Amino-cephalosporansäure in 500 ml trockenem Methylendichlorid gibt man nacheinander zuerst 35.7 g (35 ml, 0.3 mol) 2.3-Cyclopentenopyridin und dann 36 ml (0.25 mol) Trimethyljodsilan. Es wird 2 Stunden unter Rückfluß erhitzt, abgekühlt und unter Rühren ein Gemisch aus 350 ml Äthanol und 50 ml Wasser zugetropft. Während des Zutropfens bildet sich ein Niederschlag, der nach Stehen über Nacht im Kühlschrank abgesaugt wird, nacheinander zweimal mit je 80 ml Isopropanol, 80 ml Aceton sowie mit 100 ml Äther gewaschen wird. Nach dem Trocknen über $P_2O_5$ i. Vak. erhält man 19.5 g (82 % d.TH) bräunliches feinkristallines Produkt vom Zers.punkt 160 - 165°C.

$C_{16}H_{17}N_3O_3S$ x HJ x $H_2O$ (477.3)

| | | | | | |
|---|---|---|---|---|---|
| Ber. | C 40.26 | H 4.22 | J 26.59 | N 8.80 | S 6.72 % |
| Gef. | C 38.7 | H 4.2 | J 26.6 | N 8.5 | S 6.4 % |

$I_R$ (KBr): 1785 $cm^{-1}$ (Lactam-CO)

$^1H$-NMR ($CF_3CO_2D$): $\delta$ = 2.3 - 2.8 (m, 2H, Cyclopenten-H); 3.1 - 3.9 (m, 6H, 4 Cyclopenten-H und $SCH_2$); 5.3 - 6.3 (m, 4H, $CH_2Py$ und 2 Lactam-H); 7.6 - 8.8 ppm (m, 3H, Py).

Variante 2

Zu 43.8 g (0.30 mol) Hexamethyldisilan von 70 - 75°C werden portionsweise 63.5 g (0.5 mol) Jod zugegeben und die Lösung nach der Zugabe 1 Stunde unter Rückfluß erhitzt. Es wird gekühlt, mit 1l Methylendichlorid verdünnt und 71 ml (0.6 mol) 2.3-Cyclopentenopyridin und dann 27.2 g (0.1 mol) 7-Amino-cephalosporansäure auf einmal zugegeben. Die Mischung wird 2 Stunden unter Rückfluß erhitzt, dann auf 0 - 5° abgekühlt und wie vorstehend (Variante 1) beschrieben aufgearbeitet. Man erhält 39.6 g (83 % d.Th) hellbraune Kristalle. Die Verbindung ist in allen Eigenschaften mit der vorstehend beschriebenen identisch.

b) 7-[2-(2-Aminothiazol-4-yl)-2-syn-methoxyimino-acetamido]-3-[2,3-cyclopenteno-1-pyridinio)methyl]-ceph-3-em-4-carboxylat-dihydrojodid

Aus 2 g (10 mmol) 2-(2-Amino-1.3-thiazol-4-yl)-2-syn-methoxyiminoessigsäure, 1.7 g (11 mmol) 1-Hydroxybenzotriazolhydrat und 2.3 g (11 mmol) Dicyclohexylcarbodiimid in 30 ml N,N-Dimethylformamid wird eine Lösung des Aktivesters bereitet. Nach 3 Stunden Rühren bei Raumtemperatur wird vom Dicyclohexylharnstoff filtriert und die Lösung tropfenweise bei 0 - 5° zu einer Lösung von 2.4 g (5 mmol) des Hydrojodids der Stufe a und 0.4 ml (5 mmol) Pyridin in 5 ml Wasser und 40 ml N'N-Dimethylformamid zugegeben. Nach 17 Stunden bei Raumtemperatur wird das Lösungmittel i. Vak. entfernt und der Rückstand mit 20 ml Wasser digeriert. Von wenig Ungelöstem wird filtriert, zum Filtrat eine Lösung von 3.3 g (20 mmol) Kaliumjodid in 10 ml 2n HCl gegeben und der gebildete Niederschlag nach Stehen über Nacht im Kühlschrank abgesaugt. Es wird

mit wenig Eiswasser gewaschen und über $P_2O_5$ getrocknet. Ausbeute 1.9 g (48 % d.Th) hellgelbe Kristalle. Die Verbindung ist in allen Eigenschaften mit der von Beispiel 1 identisch.

Beispiel 3

7-[2-(2-Aminothiazol-4-yl)-2-syn-methoxyimino-acetamido]-3-[(2,3-cyclopenteno-1-pyridinio)methyl]-ceph-3-em-4-carboxylat-Monohydrojodid

1.1 g (5.5 mmol) 2-(2-Amino-1,3-thiazol-4-yl)-2-syn-methoxyiminoessigsäure, 0.9 g (5.9 mmol) 1-Hydroxybenzotriazolhydrat und 1.2 g (5.8 mmol) Dicyclohexylcarbodiimid in 50 ml N,N-Dimethyl-formamid werden 4 Stunden bei Raumtemperatur gerührt. Vom Dicyclohexylharnstoff wird filtriert und die Lösung des Aktivesters auf 0°C gekühlt. Es werden sodann 2,33 g (4.9 mmol) 7-Amino-3-(2,3-cyclopenteno-1-pyridinio)methyl]-ceph-3-em-4-carboxylat-Hydrojodid (Beispiel 2a) und 2.5 ml Wasser zugegeben und die Mischung 17 Stunden bei Raumtemperatur gerührt. Von wenig Ungelöstem wird filtriert und das Filtrat am Rotationsvakuumverdampfer vom Lösungsmittel befreit. Der ölige Rückstand wird mit 30 ml Äthanol vermischt, der gebildete Niederschlag abgesaugt, mit Äthanol mehrmals gewaschen und im Vakuum getrocknet.

Ausbeute: 2.7 g (86 % d.Th.)

$C_{22}H_{22}N_6O_5S_2$ x HJ

| | | | | | |
|---|---|---|---|---|---|
| Ber. | C 41.13 | H 3.61 | N 13.08 | S 9.98 | J 19.75 % |
| Gef. | C 40.6 | 3.9 | 12.5 | 10.5 | 16.8 % |

$^1$H-NMR ($CF_3CO_2D$): $\delta$ = 2.3 - 2.85 (m, 2H, Cyclopenten-H); 3.15 - 3.95 (m, 6H, 4 Cyclopenten-H und $SCH_2$); 4.42 (s, 3H, $OCH_3$); 5.2 - 6.2 (m, 4H, $CH_2$Py und 2 Lactam-H); 8.13 (s, 1H, Thiazol); 7.65 - 9.0 ppm (m, 3H, Py).

## Beispiel 4

**7-[2-(5-Amino-1,2,4-thiadiazol-3-yl)-2-syn-methoxyimino-acetamido]-3-[(2,3-cyclopenteno-1-pyridinio)methyl]-ceph-3-em-4-carboxylat**

0,2 g (1 mmol) 2-(5-Amino-1,2,4-thiadiazol-3-yl)-2-syn-methoxy-iminoessigsäure, 140 mg (1,04 mmol) 1-Hydroxybenzotriazolhydrat und 206 mg (1 mmol) Dicyclohexylcarbodiimid in 3 ml N,N-Dimethyl-formamid werden 2,5 Stunden bei Raumtemperatur gerührt. Die Reaktionslösung wird filtriert, der Dicyclohexylharnstoff mit 0,5 ml DMFA gewaschen. Zum Filtrat wird eine Lösung von 365 mg (1,1 mmol) 7-Amino-3-[(2,3-cyclopenteno-1-pyridinio)methyl]-ceph-3-em-4-carboxylat in 4 ml N,N-Dimethylformamid und 0,4 ml Wasser gegeben und die Mischung 3 Stunden bei Raumtemperatur gerührt. Das Lösungsmittel wird am Rotationsvakuumverdampfer entfernt, der Rückstand in 5 ml Wasser gelöst und über eine Lobar-B-Kieselgel-Säule (Fa. Merck, Darmstadt, Art. 10401) mit Aceton : Wasser (2 : 1) chromatographiert. Die Produktfraktionen werden eingeengt und gefriergetrocknet.

Ausbeute: 284 mg (55 % d. Th.) farbloses amorphes Produkt
IR (KBr): 1770 $cm^{-1}$ (Lactam-CO)

$^{1}$H-NMR ($CF_3CO_2D$): $\delta$ = 2,25 - 2,85 (m, 2H, Cyclopenten-H); 3,1 - 4,05 (m, 6H, 4 Cyclopenten-H und $SCH_2$), 4,30 (s, 3H, $OCH_3$); 5,2 - 6,2 (m, 4H, $CH_2Py$ und 2 Lactam-H); 7,66 - 8,0 (m, 1 Py-H), 8,16 - 8,7 ppm (m, 2 H, Py).

In Analogie zu Beispiel 2a werden die folgenden Verbindungen der allgemeinen Formel III' aus 7-Aminocephalosporansäure

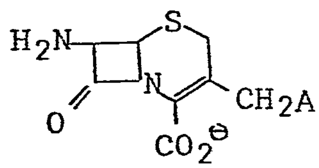 III'

und den entsprechenden, dem Rest A zugrundeliegenden Basen, erhalten.

Tabelle 1: Verbindungen 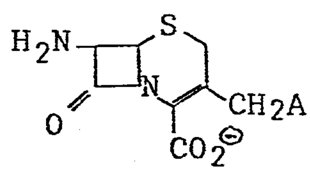

| Beisp. x) | A | Ausbeute % d.Th | $^{1}$H-NMR in $CF_3CO_2D$: $\delta$ (ppm) |
|---|---|---|---|
| I | 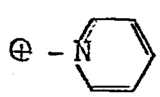 | 36 | 3.3 - 3.75 (AB, 2H, $SCH_2$); 5.3 - 6.6 (m, 4H, $CH_2Py$ und 2 Lactam-H); 7.9 - 9.45 (m, 5H, Py). |
| II | 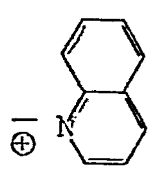 | 16 | 3.4 - 3.9 (AB, 2H, $SCH_2$); 5.2 - 6.4 (m, 4H, $CH_2Py$ und 2 Lactam-H); 7.9 - 8.8 (m, 5 Chinolin-H); 8.85 - 9.2 (m, 2 Chinolin-H). |

| x) Beisp. | A | Ausbeute % d. Th. | $^1$H-NMR in $CF_3CO_2D$: $\delta$ (ppm) |
|---|---|---|---|
| III | 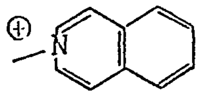 | 35 | 3.5 - 4.0 (AB, 2H, $SCH_2$); 5.1 - 6.6 (m, 4H, $CH_2Py$ und 2 Lactam-H); 7.9 - 8.8 (m, 6 Isochinolin-H); 9.8 - 9.95 (bs, 1 Isochinolin-H). |
| IV | 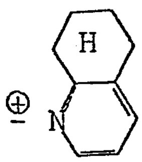 | 38 | 1.7 - 2.4 (m, 4H, Cyclohexen-H); 2.7 - 3.95 (m, 6H, 4 Cyclohexen-H und $SCH_2$); 5.35 - 6.25 (m, 4H, $CH_2Py$ und 2 Lactam-H); 7.75 - 8.65 (m, 3H, Py). |
| V | 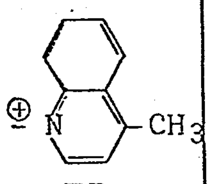 x HJ | 53 | 3.15 (s, 3H, $CH_3$); 3.2 - 4.0 (AB, 2H, $SCH_2$); 5.1 - 6.8 (m, 4H, $CH_2Py$ und 2 Lactam-H); 7.8 - 9.3 (m, 6H, Chinolin). |
| VI | 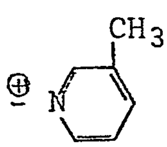 x HJ | 67 | 2.69 (s, 3H, $CH_3$); 3.66 (s, 2H, $SCH_2$); 5.25 - 6.39 (m, 4H, $CH_2Py$ und 2Lactam-H); 7.83 - 9.03 (m, 4Py-H). |

x) Die Verbindungen der Beispiele I - IV werden aus den rohen Hydrojodidsalzen durch Chromatographie über Kieselgel in amorpher Form erhalten, die Verbindungen der Beispiele V und VI direkt als Hydrojodidsalze isoliert.

Beispiel 5

7-/2-(2-Aminothiazol-4-yl)-2-syn-methoxyimino-acetamido7-3-/(2,3-cyclopenteno-1-pyridinio)methyl7-ceph-3-em-4-carboxylat

Zu einer auf 5°gekühlten Lösung von 1.02 g (2.5 mmol) 7-Amino-3-/(2,3-cyclopenteno-1-pyridinio)methyl7-ceph-3-em-4-carboxylat-Dihydrochlorid in 12,5 ml N,N-Dimethylformamid und 1,25 ml Wasser werden 1,05 g (3 mmol) 2-(2-Amino-1,3-thiazol-4-yl)-2-syn-methoxyimino-thioessigsäure-S-2-benzothiazolylester gegeben und 3 Stunden bei 5 - 10°C gerührt. Das Lösungsmittel wird im Vakuum entfernt, der Rückstand in 5 ml Wasser gelöst, mit Natriumbicarbonat auf pH 6 gestellt und die Lösung über Kieselgel (Lobar C-Säule, Fa. Merck, Art. 10402) mit Aceton : Wasser (2 : 1) chromatographiert. Die Produktfraktionen werden gefriergetrocknet und man erhält 0.86 g (66 % d. Th.) hellgelbes Produkt

$^1$H-NMR ($CF_3CO_2D$): $\delta$ = 2.40 - 2.75 (m, 2H, Cyclopenten-H); 3.22 - 4.23 (m, 6H, 4 Cyclopenten-H und $SCH_2$); 4.26 (s, 3H, $OCH_3$); 5.25 - 6.36 (m, 4H, $CH_2$Py und 2 Lactam-H); 7.38 (s, 1H, Thiazol); 7.66 - 8.58 ppm (m, 3H, Py)

In Analogie zu Beispiel 5 werden die folgenden Verbindungen der allgemeinen Formel I' aus den Verbindungen der Beispiele I - VI (Tabelle 1) und dem 2-Mercaptobenzthiazolaktivester der 2-(2-Amino-1,3-thiazol-4-yl)-2-syn-methoxyimino-essigsäure erhalten.

Tabelle 2:

Verbindungen

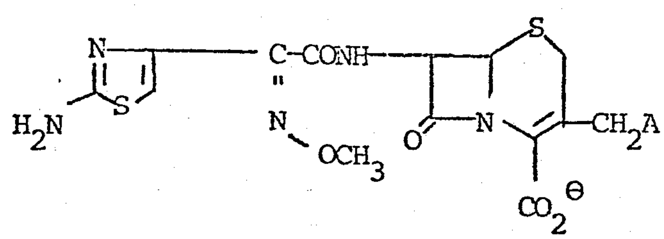

I

| Beisp. | A (Beispiel-Nr. der Ausgangsverbindung) | Ausbeute % d.Th. | $^1$H-NMR in $CF_3CO_2D$: δ (ppm) |
|---|---|---|---|
| 6 | 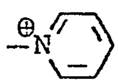 (I) | 71 | 3.52 und 3.96 (AB, J = 19Hz, 2H, $SCH_2$); 4.26 (s, 3H, $OCH_3$); 5.2 - 6.45 (m, 4H, $CH_2$Py und 2-Lactam-H); 7.43 (s, Thiazol-H); 7.9 - 9.2 (m, 5H, Py). |
| 7 | 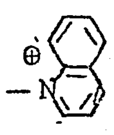 (II) | 62 | 3.40 und 3.80 (AB,J = 19Hz, 2H, $SCH_2$); 4.21 (s, 3H, $OCH_3$); 5.30 - 6.50 (m, 4H, 3-$CH_2$ und 2 Lactam-H mit je 1 d bei 5.41 und 6.10, J = 5Hz, $C_6$ bzw. $C_7$-H); 7.42 (s, 1H, Thiazol); 7.95 - 8.65 (m, 5H, Chinolin-H); 8.95 - 9.40 ppm (m, 2H, Chinolin-H) |
| 8 | 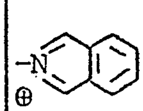 (III) | 58 | 3.45 und 3.93 (AB, J= 18 Hz, 2H, $SCH_2$); 4.21 (s, 3H, $OCH_3$); 5.25 - 6.50 (m, 4H, 3-$CH_2$ und 2 Lactam-H); 7.41 (s, 1H, |

| Beisp. | A (Beispiel-Nr. der Ausgangsverbindung) | Ausbeute % d. Th. | $^{1}$H-NMR in $CF_3CO_2D$: δ (ppm) |
|---|---|---|---|
| | | | Thiazol); 7.95 - 8.80 (m, 6H, Isochinolin-H); 9.79 ppm (bs, 1H, Isochinolin-H) |
| 9 | 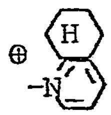 (IV) | 66 | 1.7 - 2.4 (m, 4H, Cyclohexen-H); 2.7 - 3.5 (m, 4H, Cyclohexen-H); 3.50 und 3.70 (AB, J = 19Hz, 2 H, $SCH_2$); 4.25 (s, 3H, $OCH_3$); 5.38 (d, J = 5Hz, $C_6$-H); 5.55 und 5.80 (AB, 2H, $CH_2$Py); 6.08 (d, J = 5Hz, $C_7$-H); 7.39 (s, 1H, Thiazol); 7.65 - 8.58 (m, 3H, Py) |
| 10 | 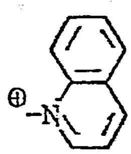 (V) | 61 | 3.15 (s, 3H, $CH_3$); 3.3 und 3.7 (AB, 2H, $SCH_2$); 4.25 (s, 3H, $OCH_3$); 5.1 - 6.7 (m, 4H, $CH_2$Py und 2 Lactam-H); 7.4 (s, 1H, Thiazol); 7.8 - 9.2 (m, 6H, Lepidin) |
| 11 | 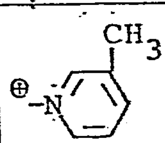 (VI) | 72 | 2.60 (s, 3H, $PyCH_3$); 3.63 und 3.86 (AB, J = 19Hz, 2H, $SCH_2$); 4.24 (s, 3H, $OCH_3$); 5.15 - 6.55 (m, 4H, $CH_2$Py und 2 Lactam-H); 7.42 (s, 1H; Thiazol); 7.75 - 9.05 (m, 4H, Py). |

Patentansprüche:

1. Verfahren zur Herstellung von Cephemverbindungen der allgemeinen Formel I,

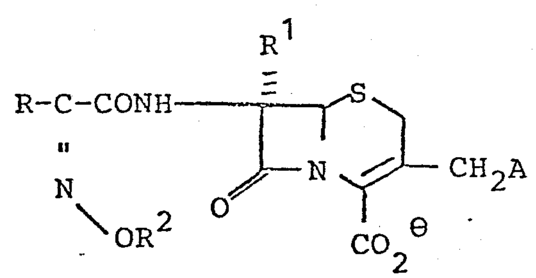

(I)

worin R einen Thiazolylrest

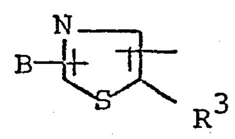

oder einen 1,2,4-Thiadiazolylrest

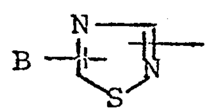

bedeutet, in denen für $R^3$ Wasserstoff oder Halogen und B für eine gegebenenfalls substituierte Aminogruppe steht,

und worin

$R^1$ Wasserstoff oder Methoxy

$R^2$ Wasserstoff, gegebenenfalls substituiertes $C_1$-$C_6$-Alkyl, gegebenenfalls substituiertes $C_2$-$C_6$-Alkenyl, $C_2$-$C_6$-Alkinyl, $C_3$-$C_7$-Cycloalkyl, $C_3$-$C_7$-Cycloalkyl-$C_1$-$C_6$-alkyl, $C_4$-$C_7$-Cycloalkenyl,

die Gruppe $(CH_2)_n \underset{R^5}{\overset{R^4}{(C)}}_m R^6$, worin m und n jeweils

für 0 oder 1 steht,

$R^4$ und $R^5$ gleich oder verschieden sein können und Wasserstoff, Aryl oder eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden, wobei die $C_1$-$C_4$-Alkyl- und $C_3$-$C_7$-Cycloalkylidengruppe noch weiter ein- oder mehrfach substituiert sein können,

$R^6$ eine COOH-, CN- oder $CONH_2$-Gruppe, wobei letztere am Stickstoff ein oder zweimal substituiert sein kann,

A einen Chinolinium 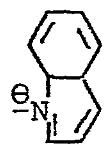 oder einen Isochinoliniumrest 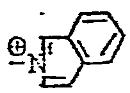, die jeweils auch ein- oder mehrfach, gleich oder verschieden substituiert sein können durch gegebenenfalls substituiertes $C_1$-$C_6$-Alkyl, $C_1$-$C_6$-Alkoxy, Halogen, Trifluormethyl oder Hydroxy, oder einen Phenanthridiniumrest, oder einen

Pyridiniumrest 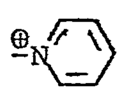, der ein- oder mehrfach, gleich oder verschieden substituiert sein kann durch gegebenenfalls substituiertes $C_1$-$C_6$-Alkyl, wobei 2 orthoständige Alkylgruppen auch zu einem gegebenenfalls substituierten Di- bis Decamethylen-Ring geschlossen sein können, in dem ein Ring-C-Atom durch ein Heteroatom ersetzt sein kann und weiterhin auch noch eine oder zwei Doppelbindungen enthalten sein können; durch gegebenenfalls substituiertes $C_2$-$C_6$-Alkenyl, durch $C_2$-$C_6$-Alkinyl, durch $C_3$-$C_7$-Cycloalkyl oder $C_3$-$C_7$-Cycloalkyl-methyl, wobei in beiden Substituenten der Ring auch substituiert sein kann; durch $C_4$-$C_7$-Cyclo-

alkenyl, durch gegebenenfalls substituiertes $C_1$-$C_6$-Alkoxy, durch $C_2$-$C_6$-Alkenyloxy oder $C_2$-$C_6$-Alkinyloxy, durch Halogen, Trifluormethyl oder Hydroxy, durch gegebenenfalls substituiertes Phenyl, Benzyl oder Heteroaryl,

durch Formyl oder ketalisiertes Formyl,

durch gegebenenfalls substituiertes $C_1$-$C_6$-Alkylcarbonyl, das auch in ketalisierter Form vorliegen kann,

durch Arylcarbonyl,

durch Carbamoyl,

bedeutet und in der die $R^2$O-Gruppe in syn-Position steht, dadurch gekennzeichnet, daß man eine Verbindung der allgemeinen Formel II,

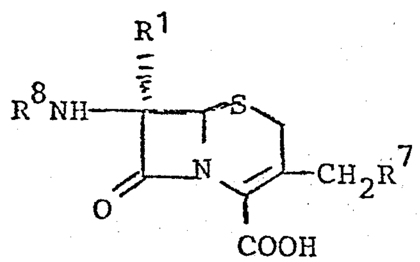

(II)

worin $R^1$ die in Formel I genannte Bedeutung hat

$R^7$ eine durch diejenige Base, die den Resten A der Formel I entspricht, austauschbare Gruppe bedeutet, und

$R^8$ für Wasserstoff oder eine Aminoschutzgruppe steht, mit der Base, die dem in Formel I definierten Rest A zugrunde liegt und in Gegenwart von Tri-$C_1$-$C_4$-alkyl-jodsilan umsetzt unter Bildung der Verbindung der allgemeinen Formel III,

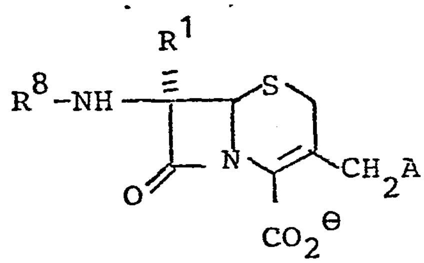

(III)

in der $R^1$, $R^8$ und A die oben genannte Bedeutung haben und

a) eine gegebenenfalls vorhandene Aminoschutzgruppe abspaltet und

b) die Verbindung III, worin $R^8$ Wasserstoff bedeutet, entweder als solche oder in Form eines reaktionsfähigen Derivates mit einer 2-syn-Oxyiminoessigsäure der allgemeinen Formel IV,

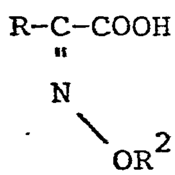

(IV)

worin R und $R^2$ die genannte Bedeutung besitzen, oder mit einem an der Carbonylgruppe aktivierten Derivat dieser Verbindung umsetzt.

2. Verfahren nach Anspruch 1, dadurch gekennzeichnet, daß das Tri-$C_1$-$C_4$-alkyljodsilan Trimethyl- oder Triethyljodsilan ist.